# EUROPEAN PATENT SPECIFICATION

(11) **EP 4 362 819 B1**
(45) Date of publication and mention of the grant of the patent: **12.08.2026**
(21) Application number: 21746256.3
(22) Date of filing: 30.06.2021
(51) Int. Cl.: A61B 17/12

(54) **VASCULAR OCCLUSION DEVICES COMPRISING OPEN STRUCTURAL COMPONENTS AND SEALED MEMBRANES**
VASKULÄRE OKKLUSIONSVORRICHTUNGEN MIT OFFENEN STRUKTURELLEN KOMPONENTEN UND ABGEDICHTETEN MEMBRANEN
DISPOSITIFS D'OCCLUSION VASCULAIRE COMPRENANT DES COMPOSANTS STRUCTURAUX OUVERTS ET DES MEMBRANES SCELLÉES

(43) Date of publication of application: 08.05.2024
(60) Divisional application: 24197556.4 / 4 450 004
(73) Proprietor: Bard Peripheral Vascular, Inc., Franklin Lakes, NJ 07417 (US)
(72) Inventor: HILL, Art, Tempe, Arizona 85281 (US)
(74) Representative: Hoffmann Eitle
(86) International application number: PCT/US2021/039820
(87) International publication number: WO 2023/277899

(56) References cited:
- US-A1- 2016 228 128
- US-A1- 2019 133 601

## Description

### TECHNICAL FIELD

The present specification generally relates to vascular occlusion devices.

### BACKGROUND

Vascular occlusion or embolization devices are intravascular implants that are intended to occlude blood flow in percutaneous interventions. For example, a vascular occlusion device may be positioned to control hemorrhaging due to aneurysms, certain tumors, and arteriovenous malformations. Vascular occlusion devices may also be positioned to block blood vessels providing flow to certain types of tumors. Existing embolization devices may rely on a complex network of components (e.g., a fiber mesh and occluding membrane) to occlude a vessel. Such devices have unnecessarily complex fabrication processes and, as a result, are costly to produce.
US 2019133601 A1 discloses a vascular occlusion device having an expandable frame that carries a membrane. The membrane can include a tubular portion configured to transition between an open configuration in which the tubular portion is configured to receive a guidewire and a closed configuration in which the tubular portion is configured to occlude blood flow.
US 2016228128 A1 discloses vascular occlusion devices.

### SUMMARY

According to the present invention, there is provided a vascular occlusion device according to claim 1.
The dependent claims define preferred embodiments.

Embodiments of the present disclosure are directed to improvements over the above limitations by providing vascular occlusion devices that occlude vessels via sintered seals of polymeric material. By relying on sintered seals as opposed to a complex network of fibers like in existing occlusion devices, the vascular occlusion devices may be produced using more efficient production processes.

In one embodiment, a vascular occlusion device includes a structural component comprising an axis and a membrane that contacts the structural component and is constructed of a polymeric material. The membrane includes a first end and a second end. At least one of the first end and the second end extends axially beyond the structural component along the axis and includes a sintered seal that closes an end of the structural component.

In another embodiment, a vascular occlusion device includes a structural component and an expanded polytetrafluoroethylene (ePTFE) membrane surrounding the structural component. The ePTFE membrane includes a substantially cylindrical-shaped tube having a wall thickness and a sintered end comprising melted and re-solidified ePTFE. A distance separates sintered end and an end of the structural component.

In yet another embodiment, a method of forming a vascular occlusion device, the method includes positioning a structural component in an expanded polytetrafluoroethylene (ePTFE) membrane such that an end of the ePTFE membrane extends beyond an end of the structural component. The method also includes sintering the end of the ePTFE membrane to seal off a cavity delineated by the ePTFE membrane.

In yet another embodiment, a method of occluding a vessel includes guiding a vascular occlusion device to an occlusion position within a catheter and removing the catheter. The vascular occlusion device includes a structural component comprising an axis; and a membrane that contacts the structural component and is constructed of a polymeric material. The membrane includes a first end and a second end. At least one of the first end and the second end extends axially beyond the structural component along the axis and includes a sintered seal. The method also includes expanding the structural component such that the occlusion device contacts a vessel wall and occludes the vessel via the sintered seal.

These and additional features provided by the embodiments described herein will be more fully understood in view of the following detailed description, in conjunction with the drawings.

### BRIEF DESCRIPTION OF THE DRAWINGS

The embodiments set forth in the drawings are illustrative and exemplary in nature and not intended to limit the subject matter defined by the claims. The following detailed description of the illustrative embodiments can be understood when read in conjunction with the following drawings, where like structure is indicated with like reference numerals and in which:
FIG. 1A schematically depicts a vascular occlusion device, according to one or more embodiments described herein;
FIG. 1B schematically depicts a view of a distal end of the vascular occlusion device of FIG. 1A, according to one or more embodiments described herein;
FIG. 1C schematically depicts a cross-sectional view of the vascular occlusion device through the line I-I of FIG. 1A, according to one or more embodiments described herein;
FIG. 2A schematically a vascular occlusion device in an unexpanded device as delivered to a vessel, according to one or more embodiments described herein;
FIG. 2B schematically depicts the vascular occlusion device of FIG. 2A in an expanded state occluding a vessel via contact with vessel walls, according to one or more embodiments described herein;
FIG. 3 schematically depicts a vascular occlusion device including a structural component with an end structure, according to one or more embodiments described herein;
FIG. 4 schematically depicts a vascular occlusion device including a stent graft as a structural component, according to one or more embodiments described herein;
FIG. 5 depicts a flow diagram of a method of fabricating a vascular occlusion device, according to one or more embodiments described herein; and
FIG. 6 depicts a flow diagram of a method of occluding a vessel using a sintered seal of a vascular occlusion device, according to one or more embodiments described herein.

### DETAILED DESCRIPTION

Embodiments of the present disclosure are directed to vascular occlusion devices including structural components that support membranes including one or more sintered seals to occlude blood flow within a vessel. In embodiments, the structural component may include an open structure (e.g., include one or more open ends) that circumferentially defines a lumen extending along an axis. The membrane may include at least one end that extends axially beyond the structural component (e.g., in a direction of the axis). Different portions of the at least one end of the membrane may be sintered to one another to form a sintered seal that closes off (e.g., prevents blood flow from reaching) the lumen of the structural component. The combined structure of the membrane and structural component may occlude an entire section of the vessel to achieve embolization of the vessel. By sintering the membrane to close the lumen of the structural component, the vascular occlusion devices of the present disclosure may achieve occlusion with structural components that are less expensive and time consuming to fabricate than those used in currently existing vascular embolization devices.

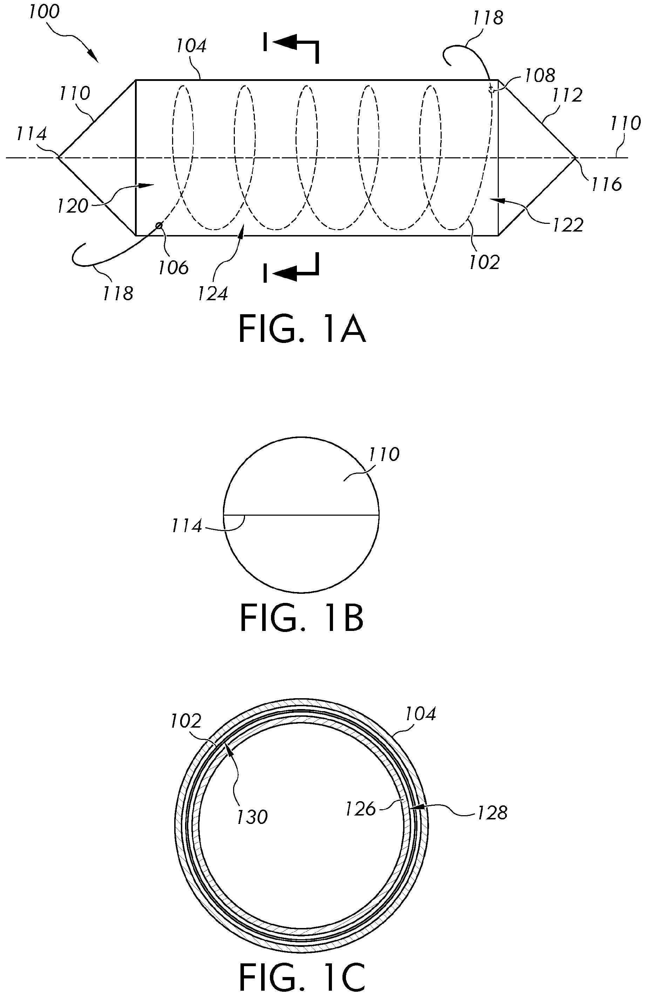

FIG. 1A schematically depicts a vascular occlusion device 100 according to an example embodiment. FIG. 1A schematically depicts the vascular occlusion device 100 in an expanded state. The expanded state may represent the size and configuration of the vascular occlusion device 100 in an as-deployed condition within a blood vessel. It should be understood that the vascular occlusion device 100 may include a different size and/or shape in an un-expanded state (e.g., when not deployed within a blood vessel). The vascular occlusion device 100 includes a structural component 102 and a membrane 104 that contacts the structural component 102. In embodiments, the structural component 102 is an open structure including a structural frame that circumferentially surrounds an axis A. The structural component 102 includes a first end 106 and a second end 108. In embodiments at least one of the first end 106 and the second end 108 may be open ended. For example, in the embodiment depicted in FIG. 1A, the structural component 102 includes a first open end 120 (e.g., at a distal end thereof) and a second open end 122 (e.g., at a proximal end thereof). In embodiments, the structural component 102 is free of components (e.g., fibers, mesh structures, members) that extend radially inward towards the axis from the open structure surrounding the axis A. The structural component 102 may be constructed of a biocompatible material such as nitinol. In embodiments, the structural component 102 is a coil structure patterned from a tube of a biocompatible material, for example. The structural component 102 may have a variety of different forms (e.g., a helical coil, an alternating helical coil, a tubular mesh structure, a substantially cylindrical frame comprising a plurality of axial and circumferential components) depending on the implementation. For example, in some embodiments, the structural component 102 may be a self-, balloon-, or otherwise-expandable stent or similar structure. It should also be appreciated that the structural component 102 may have a variety of sizes (e.g., both axially and radially).

The membrane 104 includes a first end portion 110 and a second end portion 112. In embodiments, at least one of the first end portion 110 and the second end portion 112 extends axially beyond the first end 106 and/or the second end 108 of the structural component 102. For example, in the embodiment depicted in FIG. 1A, both the first end portion 110 and the second end portion 112 extend axially beyond the first end 106 and the second end 108 of the structural component 102, respectively. As described herein, the non-overlapping ends of the structural component 102 and the membrane 104 may facilitate the vascular occlusion device 100 occluding flow within a vessel despite the open structure of the structural component 102, or despite the structural component 102 lacking components extending perpendicular to flow in some embodiments.

In embodiments, the membrane 104 is constructed of a suitable polymeric material. In embodiments, the membrane 104 includes a woven polymer fabric or textile. In embodiments, the membrane 104 is constructed of expanded polytetrafluoroethylene (ePTFE), polyolefin, or polyester. Constructing the membrane 104 of ePTFE may facilitate anchoring the vascular occlusion device 100 within a blood vessel. For example, the membrane 104 may include pores that facilitate blood coagulation thereon to aid in adhering the vascular occlusion device 100 to vessel walls. In embodiments, the membrane 104 is a substantially cylindrical tube-like structure that is structurally supported by the structural component 102 to circumferentially surround the axis A. While membrane 104 depicted in FIG. 1A is disposed outside (e.g., covering) the structural component 102, it should be understood that embodiments are also envisioned where the membrane 104 is disposed radially inward of the structural component (e.g., such that the structural component 102 or a cover layer disposed thereon contacts a vessel wall). In embodiments, the membrane 104 includes an axial length (e.g., in the direction of the axis A depicted in FIG. 1A) that is greater than that of the structural component 102 such that ends thereof are axially offset from the first end 106 and the second end 108 of the structural component 102. It should be understood that the axial length and radial dimensions of the membrane 104 may vary depending on the implementation and that membranes having wide variety of sizes and/or shapes are contemplated and within the scope of the present disclosure.

With reference to FIGS. 1A and 1B, the membrane 104 is depicted to include a first sintered seal 114 (e.g., at a first or distal end thereof) and a second sintered seal 116 (e.g., at a second or proximal end thereof). In embodiments, the first end portion 110 and the second end portion 112 comprise open ends of a tube-shaped body of the membrane 104 that are sintered shut at the first and second sintered seals 114 and 116. For example, the first sintered seal 114 and the second sintered seal 116 may be melted joints of the polymeric material out of which the membrane 104 is constructed. The melted joints may aid in strengthening the first and second end portions 110 and 112, as segments of the material of the membrane 104 may contact one another on either side thereof, thereby increasing the thickness of the membrane 104 at the melted joints. In embodiments, the first and second sintered seals 114 and 116 are axially offset from the first and second ends 106 and 108 of the structural component 102 (e.g., by gaps or separation distances), respectively. Such gaps may aid in forming the first and second sintered seals 114 and 118 (e.g., via application of heat from a suitable heat source) without heating the structural component 102 to a significant extent. Heating of the structural component 102 may alter the thermal treatments thereof and alter the shape of the structural component 102 (e.g., when the structural component 102 is formed from a shape memory material such as a shape memory polymer or a shape memory alloy such as nitinol).

As depicted in FIG. 1B, the melted joint of the first sintered seal 116 may extend an entirety of a distance between two opposing portions of the membrane 104. In embodiments, the second sintered seal 116 (see FIG. 1A) has a similar construction to that depicted in FIG. 1B such that, via the first sintered seal 114 and the second sintered seal 116, a cavity 124 is completely closed off from an environment external to the vascular occlusion device 100. In embodiments, the cavity 124 may be filled with a suitable gas to facilitate the vascular occlusion device 100 maintaining its shape when encountering blood flow.

With reference to FIG. 1B, in embodiments, a length of the first sintered seal 114 may correspond to a diameter of a vessel such that blood flow in the vessel is occluded by the vascular occlusion device 100. As such, the vascular occlusion device 100 may occlude the vessel via at least one of the first sintered seal 114 and the second sintered seal 116. The material of the membrane 104 closes off the first open end 120 and the second open end 122 of the structural component 102 to block blood flow therethrough. The material of the membrane 104 extends between opposing portions of the structural component 102 (e.g., at opposite ends of a diameter extending through the axis A) to prevent blood from flowing through the first open end 120 and the second open end 122. By relying on the material of the membrane 104 to occlude blood flow, the vascular occlusion device 100 does not rely on the structural component 102 to form an occlusion structure that blocks flow proximate to the axis A. Instead, the structural component 102 provides structural support to the membrane 104 around a periphery of the vascular occlusion device 100 and the material of the membrane 104 extends radially inward from the structural component 102 to prevent flow therethrough. In the embodiment depicted in FIGS. 1A-1B, for example, the first end portion 110 of the membrane 104 is not in contact with the structural component 102 and extends radially inward from the structural component 102 to occlude blood flow. Such a structure aids in reducing the complexity of the structure of the structural component 102 as compared to those contained in existing vascular occlusion devices, thereby simplifying production processes. The open structure of the structural component 102 may also aid in collapsing the vascular occlusion device 100 into an unexpanded state to facilitate delivery of the vascular occlusion device 100 into a vessel (e.g., via a catheter) and placement at a desired position.

While the embodiment depicted in FIGS. 1A and 1B includes the first and second end portions 110 and 112 having similar structures (e.g., both including sintered seals), it should be understood that alternative embodiments are contemplated and within the scope of the present disclosure. For example, embodiments are envisioned where the vascular occlusion device 100 only includes a one of the first end portion 110 and the second end portion 112 and only includes a single sintered seal. In such embodiments, the membrane 104 may include an open end (e.g., on an end opposite to the direction of the blood flow that the vascular occlusion device 100 is used to occlude) to provide access to the cavity 124 from the open end. Such an open end may aid in deployment of the vascular occlusion device 100 by allowing components of a catheter assembly to be inserted into the vascular occlusion device 100 during deployment.

Alternative structures for the first end portion 110 and/or the second end portion 112 are also contemplated and within the scope of the present disclosure. In the depicted embodiment, for example, the first end portion 110 is a symmetrical structure about the first sintered seal 114, with two equally-sized portions of the membrane 104 being pressed inwards and joined together at the first sintered seal 114. Embodiments are envisioned where the first and second end portions 110 and 112 are asymmetrical (e.g., where portions of the membrane 104 of differing lengths are pressed inward to form the first and second sintered seals 114 and 116). Moreover, embodiments are also envisioned where the first sintered seal 114 and/or the second sintered seal 116 are non-linear in shape. For example, in embodiments, the first sintered seal 114 may form a point, with the first end portion 110 including a conical shape. In embodiments, the first end portion 110 may have a geometry that differs from the second end portion 112 (e.g., the first sintered seal 114 may have a linear shape as depicted in FIG. 1B, while the second sintered seal 116 may be a point).

Alternative locations for the first sintered seal 114 and the second sintered seal 116 are also envisioned. For example, in embodiments, the first sintered seal 114 may lie along an outer peripheral surface of the vascular occlusion device 100 (e.g., along the membrane 104 outside of the first end portion 110). For example, a portion of the first end portion 110 may be folded over the first open end 120 of the structural component 102 and the first sintered seal 114 may be formed at an interface between the folded portion and the tube-like body of the membrane 104. In such embodiments, the first end portion 110 may extend perpendicular to the axis A and the first sintered seal 114 may not directly contact the blood flow in the vessel.

In the embodiment depicted in FIGS. 1A and 1B, the membrane 104 is formed from a single integrated body. For example, in embodiments, the membrane 104, the first end portion 110, and the second end portion 112 are formed from an ePTFE tube. Embodiments where the membrane 104 is constructed from a plurality of separate components are also contemplated and within the scope of the present disclosure. For example, in embodiments, the first and second end portions 110 and 112 may include separate pieces of polymeric material that are welded to the membrane 104. In such embodiments, the first and second sintered seals 114 and 116 may include shapes corresponding to a circumferential shape of the membrane 104 and structural component 102. For example, the first and second sintered seals 114 and 116 may be substantially circular-shaped and extend around a circumference of the membrane 104 at ends thereof to cover the first and second open ends 120 and 122. The first and second end portions 110 and 112 may also be constructed of a different material than the remainder of the membrane 104. In an example, the first and second end portions 110 and 112 are constructed of an elastic polymeric material such as urethane and the remainder of the membrane 104 is constructed from ePTFE, though other combinations are contemplated and possible. Embodiments are also envisioned where the portion of the membrane 104 circumferentially surrounding the axis A (e.g., contacting or extending parallel to a wall of a vessel) is constructed of a plurality of different components (e.g., a plurality of different ePTFE tubes may be sintered or otherwise coupled to one another in an overlapping fashion to form the membrane 104).

With reference to FIG. 1A, in embodiments, the vascular occlusion device 100 includes one or more anchors 118 extending radially outward from the structural component 102. In embodiments, the one or more anchors 118 are integral with the structural component 102 (e.g., formed from the same piece of material) and outward from the remainder of the structure component 102. For example, the one or more anchors 118 may extend radially outward from the membrane 104. In the embodiment depicted in FIG. 1A, the one or more anchors 118 includes a first anchor extending from the first end 106 of the structural component 102 and a second anchor extending from the second end 108 of the structural component 102. In embodiments, the one or more anchors 118 may extend through different portions of the membrane 104 disposed proximate to the first end 106 and the second end 108, respectively. As the structural component 102 expands to the configuration depicted in FIG. 1A (e.g., via the shape memory effect when the structural component 102 is formed of nitinol) when deployed in a vessel, the one or more anchors 118 may extend into walls of the vessel and prevent the vascular occlusion device 100 from migrating from a desired location. Embodiments are also envisioned where the one or more anchors are formed separately from the structural component 102 and attached thereto via a suitable connection method (e.g., via welding, brazing, or the like).

FIG. 1C schematically depicts a cross-sectional view of the vascular occlusion device 100 through the line I-I of FIG. 1A. In embodiments, the vascular occlusion device 100 comprises an inner liner 126 disposed radially inward of the structural component 102. In embodiments, an exterior surface 128 of the inner liner 126 contacts an interior surface 130 of the structural component 102. The inner liner 126 may be a substantially cylindrical-shaped tube having a wall thickness. In embodiment, the inner liner 126 includes a structure that is similar to that of the membrane 104 described herein. For example, in embodiments, the inner liner 126 may include an ePTFE tube. That is, the inner liner 126 and the membrane 104 may be constructed of the same material. In embodiments, the inner liner 126 and the membrane 104 may be formed of different materials.

In embodiments, the structural component 102 is disposed in an annular space between the inner liner 126 and the membrane 104. The inner liner 126 may include an axial length corresponding to an axial distance between the first and second ends 106 and 108 of the structural component 102. In some embodiments, the inner liner 126 includes an open-ended structure (e.g., lacks structures corresponding to the first and second end portions 110 and 112 described herein with respect to FIGS. 1A and 1B). In some embodiments, the inner liner 126 comprises end portions that are closed via sintered seals similar to the first and second end portions 110 and 112 of the membrane 104. It should be appreciated that the inner liner 126 is optional and embodiments are envisioned that do not include the inner liner 126.

In some embodiments, the first and second end portions 110 and 112 of the membrane 104 described herein with respect to FIGS. 1A and 1B are components of the inner liner 126. In such embodiments, the vascular occlusion device 100 may lack the membrane 104.

The relative radial positioning of the structural component 102 and the polymer-based material (e.g., either of the membrane 104 or the inner line 126) that is sintered to occlude a vessel may vary depending on the implementation. Any vascular occlusion device where a segment of polymer-based material is adhered to another segment of polymer-based material to occlude a vessel is within the scope of the present disclosure. As used herein, the term "sintered seal" is used to describe instances where portions of polymeric material are joined together using a suitable adhesion technique. Sintered seals may be produced by welding, heating, pressure, or any combination thereof. Moreover, the terms "sintered seal" or "sintered end," as used herein, may encompass embodiments where portions of polymer material are attached to one another via another suitable technique (e.g., a biocompatible adhesive).

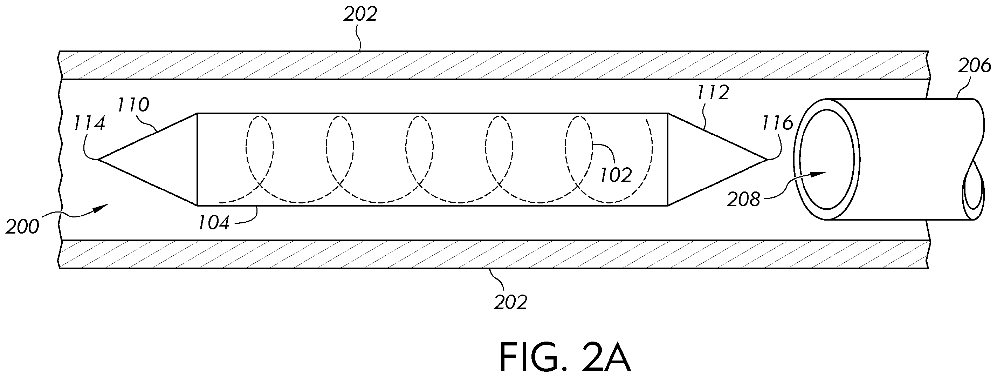

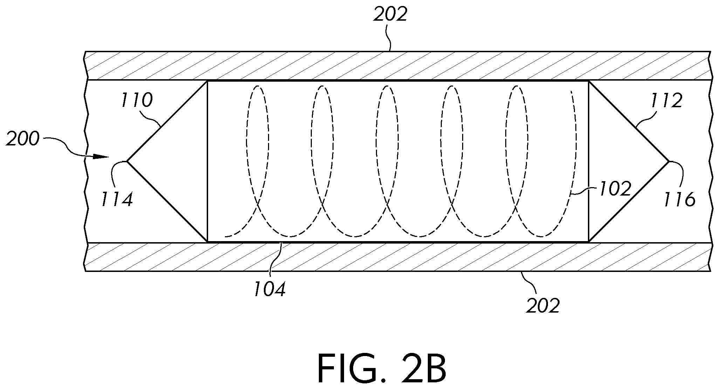

FIG. 2A schematically depicts a vascular occlusion device 200 disposed in a vessel in an unexpanded state. The vascular occlusion device 200 depicted in FIG. 2 may be similar in structure to the vascular occlusion device 100 described herein with respect to FIGS. 1A-1C, with the exception that the vascular occlusion device 200 does not include the one or more anchors 118. Accordingly, like reference numerals are used in FIG. 2A to indicate the incorporation of such like components. As depicted in FIG. 2A, when initially disposed inside the vessel, the structural component 102 may be radially contracted such that the vascular occlusion device 200 has a diameter than is less than that of the vessel, when in the unexpanded state. The vascular occlusion device 200 may be delivered into the vessel via a catheter 206. For example, the vascular occlusion device 200 may be advanced to a desired position via a lumen 208 of the catheter 206 and released from the catheter 206 via a suitable delivery mechanism. In embodiments, the membrane 104 comprises an inner liner and outer ePTFE liner that is sintered around the structural component 102 such that the ePTFE liners expand in conjunction with the structural component 102 once placed in the vessel. That is, and as noted above, the structural component 102 may be an expandable structure (e.g., a self- expandable, stent or coil) formed from shape-memory materially that automatically expands to the expended state once deployed, such as in response to body temperature. Though in other embodiments, it is contemplated that the structural component 102 may be expandable via some expansion actuator such as a balloon, for example. As a result, the first and second end portions 110 and 112 may expand in conjunction with the structural component 102 to occlude the vessel via the first and second sintered seals 114 and 116. In embodiments, the membrane 104 includes an axial length that is greater than the structural component 102 when the structural component is in the unexpanded state, thus causing the first and second end portions 110 and 112 to vary in shape by expanding by an amount that is dependent on axial position relative to the ends of the structural component 102 once the vascular occlusion device 200 is deployed in the vessel.

FIG. 2B depicts the vascular occlusion device 200 in an expanded state. As depicted in FIG. 2B, when the vascular occlusion device 200 is in the expanded state, the structural component 102 may apply a force (e.g., a radial force) to walls 202 of the vessel. For example, in embodiments, the structural component 102 may compress the membrane 104 against the walls 202 such that an outer peripheral surface of the membrane 104 corresponds in shape to surfaces of the walls 202. As a result, the first and second end portions 110 and 112 (e.g., via first and second sintered seals 114 and 116) occlude the vessel by filling an entirety of the space extending between the walls 202.

In some embodiments, the structural component 102 may directly contact the walls 202. In such embodiments, and as described above, the membrane 104 may be disposed radially inward of the structural component 102 (e.g., in a position corresponding to the inner liner 126 depicted in FIG. 1C). Blood flow in the vessel may contact the structural component 102 (e.g., proximate to the first and second ends 106 and 108, see FIG. 1A), but the vascular occlusion device 200 may occlude blood flow via pressure applied to the walls 202 via the structural component 102. For example, in embodiments, the structural component 102 may be configured to expand to a radial dimension larger than that of the vessel (e.g., such that different circumferential portions thereof are separated by a distance greater than a distance separating the walls 202) if left unimpeded. As a result, expansion of the structural component 102 may result in the structural component 102 applying a pressure to the walls 202 and creating a seal that blocks blood flow. In embodiments, coagulated blood may accumulate on an external surface of the membrane 104 and result in a seal being formed at the interface between the vascular occlusion device 200 and the walls 202. The first end portion 110 and the second end portion 112, by preventing blood from flowing radially inward of the structural component 102, result in the total blockage of blood flow within the vessel.

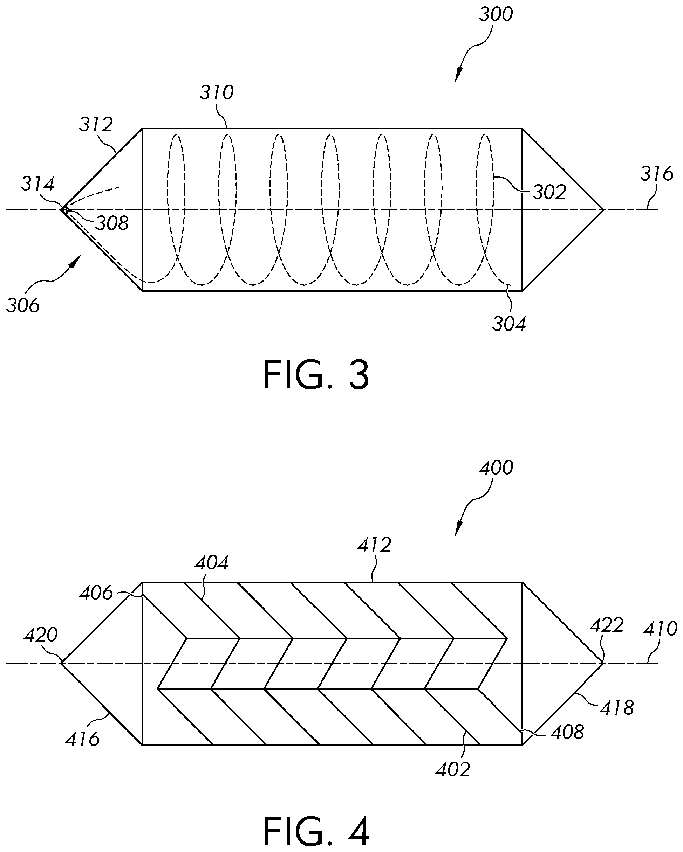

FIG. 3 schematically depicts another vascular occlusion device 300 according to another example embodiment. The vascular occlusion device 300 includes a structural component 302 and a membrane 310. In embodiments, the membrane 310 is substantially similar to the membrane 104 of the vascular occlusion device 100 described herein with respect to FIGS. 1A-1C and may include any of the structures described herein with respect to the membrane 104. For example, the membrane 310 may include a substantially cylindrical-shaped tube of a suitable polymeric material such as ePTFE. The membrane 310 is also depicted to include an end portion 312 and a sintered seal 314 (e.g., a sintered end) that may be similar in structure to the first end portion 110 and first sintered seal 114 described herein with respect to FIGS. 1A-1C.

The structural component 302 may be substantially similar to the structural component 102 of the vascular occlusion device 100 described herein with respect to FIGS. 1A and 1C (e.g., including an open structural frame such as a nitinol coil or other suitable structure). The structural component 302 is depicted to include a first end 304 and a second end 308 displaced from the first end 304 along an axis A of the vascular occlusion device 300. The structural component 302 may include an open end (e.g., free of material) proximate to the first end 304. The structural component 302 differs from the structural component 102 described herein with respect to FIGS. 1A-1C in that the second end 308 extends into an end portion 312 of the membrane 310. In embodiments, the second end 308 may include a portion of a wire from which the structural component 302 is formed, for example that is bent into a desired shape. The second end 308 may contact a sintered seal 314 formed in the end portion 312 of the membrane 310. The second end 308 may provide structural support to the end portion 312 at the sintered seal 314 and aid in reducing stress on the material of the membrane 310.

In embodiments, the second end 308 may be structured in a way that corresponds to an interior surface of the end portion 312 to provide structural support thereto at positions that are displaced from the sintered seal 314. For example, the second end 308 and the end portion 312 may be compressed and formed in the same processing step (e.g., from tubes of different respective materials) such that the second end 308 substantially corresponds in shape to the end portion 312. The structural component 302 may include a coil portion providing structural support to a body of the membrane 310 and a non-coil portion at the second end 308. The non-coil portion may extend directly from an end of the coil portion and be patterned differently (e.g., the non-coil portion at the second end 308 may include a tubular segment having a compressed end to substantially correspond in shape to the end portion 312). In embodiments, the structural component 302 may vary in structure from the end portion 312 at the second end 308 to selectively support a particular location of the end portion 312 (e.g., at the sintered seal 314, radially displaced from the sintered seal).

In the vascular occlusion device 300, the sintered seal 314 may not be separated from the structural component 302. There may be at least one point of contact between the structural component and the end portion 312 of the membrane 310 used to occlude the vessel. The at least one point of contact may increase the durability of the vascular occlusion device 300. While the second end 308 of the structural component 302 is depicted to contact the end portion 312 in FIG. 3, it should be understood that alternative portions of the structural component 302 may contact the end portion 312 in alternative embodiments. For example, in embodiments, the structural component 302 includes a support structure (not depicted) extending from a coil portion of the structural component 302 (e.g., offset from the second end 308) to contact the end portion 312. Such a support structure may have any suitable shape to support the end portion 312 in any desired manner.

Referring still to FIG. 3, the membrane 310 of the vascular occlusion device 300 is depicted to include an open end 316. The open end 316 may be devoid of the material of the membrane 310 and provide access to the interior of the vascular occlusion device 300. Such access may allow insertion of components therein to aid in deployment of the vascular occlusion device 300. In the depicted embodiment, the end portion 312 may be used to occlude blood flow and the vascular occlusion device 300 may be positioned in a vessel such that blood flow initially contacts the end portion 312. Embodiments are also envisioned where the membrane 310 includes an additional end portion (e.g., covering the open end 316 depicted in FIG. 3). The structural component 302 may contact such an additional end portion via a structure extending from the first end 304.

FIG. 4 schematically depicts another example vascular occlusion device 400. The vascular occlusion device 400 includes a structural component 402 and a membrane 412. In embodiments, the membrane 412 includes a first end portion 416 and a second end portion 418. The membrane 412 may be similar in structure to the membrane 104 described herein with respect to FIGS. 1A-1C and include first and second sintered seals 420 and 422 for enclosing an interior volume and occluding blood flow.

In the depicted embodiment of FIG. 4, the structural component 402 may be a stent graft structure. For example, in embodiments, the structural component 404 comprises a plurality of support structures 404 that are interconnected with one another to circumferentially surround an axis A of the vascular occlusion device 400. The plurality of support structures 404, for example, may include a mesh of a suitable structural support material such as stainless steel or a suitable alloy. The structural component 402 may be any suitable structure. For example, the structural component 402 may be an expandable (e.g., balloon expandable such as where only one end of the of the membrane 412 is closed, self-expandable, such as via a stent or coil formed of a shape memory material, or expandable via any suitable expansion mechanism.

Because the material of the membrane 412 occludes flow of the vessel via one or more of the first sintered seal 420 and the second sintered seal 422, a variety of open frame-like structures may be used as the structural component 402. The design of the vascular occlusion device 400 thus provides flexibility to re-purpose various existing open structures as structural supports for vascular occlusion devices. Any suitable structure that can be expanded in shape to conform to a shape a vessel may be used for a structural component of the vascular occlusion devices herein because the vascular occlusion devices described herein do not rely on an structural components extending non-parallel to the occluded blood flow to provide an occluding structure.

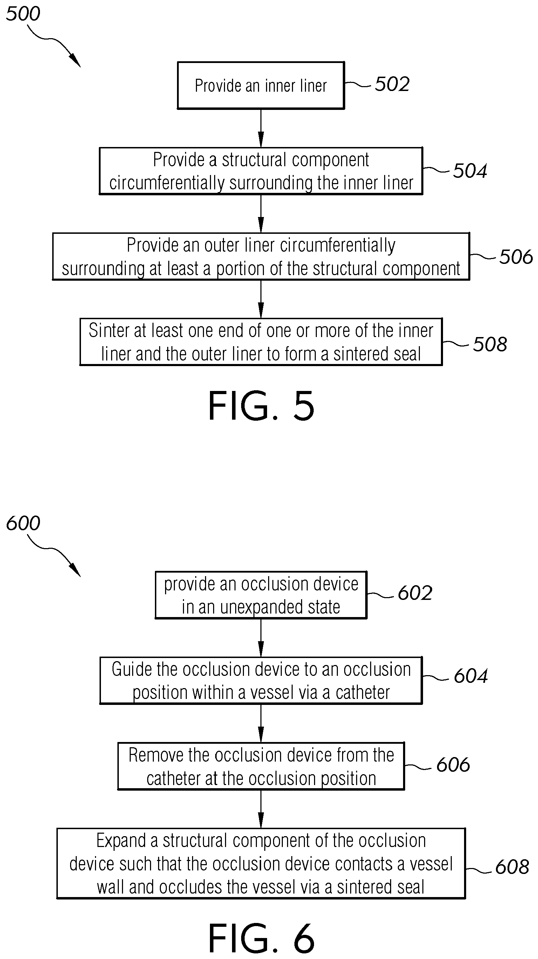

FIG. 5 depicts a flow diagram of a method 500 of fabricating a vascular occlusion device, according to an example embodiment of the present disclosure. For example, the method 500 may be performed to fabricate any of the vascular occlusion devices (e.g., the vascular occlusion devices 100, 200, 300 and 400 described herein with respect to FIGS. 1A-4) described herein. To aid in the description of the method 500, references will be made to the vascular occlusion device 100 described herein with respect to FIGS. 1A-1C. It is noted that a greater or fewer number of steps may be included, in any order, without departing from the scope of the present disclosure.

At step 502, the method 500 includes providing an inner liner. For example, as described herein with respect to FIG. 1C, the inner liner 126 of the vascular occlusion device 100 may include a tube of a suitable polymeric material. In embodiments, the inner liner 126 is formed of ePTFE. Such an inner liner 126 may be fabricated by initially fabricating a PTFE tube (e.g., via a suitable extrusion process). The PTFE tube may be heated and stretched to convert the PTFE tube into an ePTFE tube that serves as the inner liner 126. The ePTFE tube may be provided with any suitable radial dimension and length depending on a desired size of the vascular occlusion device 100. It should be appreciated that embodiments are envisioned where the method 500 does not include the step 502. At step 504, the method 500 includes providing a structural component circumferentially surrounding the inner liner. For example, in embodiments, the inner liner 126 may be provided over a mandrel, and a nitinol wire may be wound around the mandrel in a desired pattern to form the structural component 102. In embodiments, the combination of the inner liner 126 and the nitinol wire may be subjected to a heat set process to set the coil to a desired expanded state at an elevated temperature. In embodiments, the structural component 102 is formed and/or heat set prior to being placed into contact with the inner liner 126.

At step 506, the method 500 may include providing an outer liner circumferentially surrounding at least a portion of the structural component. For example, in embodiments, the membrane 104 comprises a tape of suitable polymeric material (e.g., ePTFE) that is wrapped around the structural component 102. In embodiments, the membrane 104 is a separately extruded ePTFE tube into which the inner liner 126 and structural component 102 are inserted. The structural component 102 may be positioned within the membrane 104 such that at least one end of the membrane 104 (e.g., corresponding to the first end portion 110 and/or the second end portion 112) extends axially beyond the structural component 102. In embodiments, after provision of the outer liner, the entire assembly may be placed in a sintering furnace such that the inner and outer liners are sintered together with the structural component being encapsulated within polymeric material. At step 508, the method includes sintering at least one end of one or more of the inner liner and the outer liner to form a sintered seal. For example, in the vascular occlusion device 100, both the first end portion 110 and the second end portion 112 of the membrane 104 are subjected to a secondary joining process where portions of the membrane 104 are pressed together and heated via an energy source (e.g., an ultrasonic energy beam, a laser, a torch, a thermocompression bonding apparatus) to form the first and second sintered seals 114 and 116. Axial separation between the structural component 102 and the first and second end portions 110 and 112 may beneficially avoid heat being applied to the structural component 102, which may affect the thermal behavior thereof. Pressure may be applied in any distribution to the inner our outer liners to provide sintered seals having any desired shape or form.

Referring now to FIG. 6, a method 600 of occluding a vessel via deployment of a vascular occlusion device therein is shown, according to an example embodiment of the present disclosure. It is noted that a greater or fewer number of steps may be included, in any order, without departing from the scope of the present disclosure. In an example, the method 600 may be performed to position the vascular occlusion device 200 described herein with respect to FIGS. 2A-2B at a target location within a vessel to occlude blood flow within the vessel. Accordingly, reference will be made to various components of the vascular occlusion device 200 of FIGS. 1A-1C to aid in the description of the method 600.

At step 602, an occlusion device in an unexpanded state is provided. For example, the structural component 102 of the vascular occlusion device 200 may include a shape memory component (e.g., a nitinol coil) that is in an unexpanded state below body temperature (e.g., at room temperature). As a result, a radial dimension of the vascular occlusion device 100 may be diminished, as depicted in FIG. 2A, when beneath body temperature. In embodiments, the structural component 102 is an elastic structure that is compressed into the unexpanded state (e.g., via insertion into a catheter assembly). In embodiments, the structural component 102 is in the unexpanded state by default at room temperature and is expanded within the vessel via an expanding mechanism (e.g., a balloon, shape-memory materials, etc.) associated with a delivery assembly.

At step 604, the occlusion device 200 is guided to an occlusion position within a vessel via a catheter 206. The catheter 206 may be a flexible tubing configured for traversal through one or more body vessels. The catheter 206 may be sized and shaped to be traversed through a vein of a user to the occlusion position. The catheter 206 may define a lumen 208 into which the vascular occlusion device 200 and a suitable delivery assembly (e.g., a pushing mechanism or deployment tube) are inserted. At step 608, the structural component 102 of the vascular occlusion device 200 is expanded such that the vascular occlusion device 200 contacts a vessel wall 202 and occludes the vessel via the sintered seal, as depicted in FIG. 2B. For example, the vascular occlusion device 200 may be removed from the catheter 206 via a delivery mechanism disposed therein. As a result of being removed from the catheter 206, the structural component 102 may radially expand to contact the walls of the vessel. As a result, one or more of the first end portion 110 and the second end portion 112 occludes blood flow in the vessel via the first sintered seal 114 and/or the second sintered seal 116.

It should now be understood that embodiments of the present disclosure are directed to vascular occlusion devices that occlude blood flow within a vessel via one or more sintered seals of polymer material. For example, a closure device may include a structural component and a membrane including an end portion extending axially beyond the structural component. The membrane may be formed of a suitable polymeric material such as ePTFE. The end portion may extend radially inward and contain one or more sintered seals where portions of the polymeric material are joined together to close an end of the vascular occlusion device. The closed end may serve to block blood flow within the vessel. Accordingly, since the sintered seal of polymeric material is used to occlude the blood flow, open-ended structural components may be used that are more easily fabricated than structural components associated with existing vascular occlusion devices.

It is noted that the terms "substantially" and "about" may be utilized herein to represent the inherent degree of uncertainty that may be attributed to any quantitative comparison, value, measurement, or other representation. These terms are also utilized herein to represent the degree by which a quantitative representation may vary from a stated reference without resulting in a change in the basic function of the subject matter at issue.

While particular embodiments have been illustrated and described herein, it should be understood that various other changes and modifications may be made without departing from the scope of the claimed subject matter. Moreover, although various aspects of the claimed subject matter have been described herein, such aspects need not be utilized in combination.

## Claims

1. A vascular occlusion device (100) comprising:
a structural component (102); and
an expanded polytetrafluoroethylene (ePTFE) membrane (104) surrounding the structural component (102), wherein the ePTFE membrane comprises:
a substantially cylindrical-shaped tube having a wall thickness; and
a sintered end (114, 116) comprising melted and re-solidified ePTFE, wherein there is a distance between the sintered end and an end of the structural component (102).

2. The vascular occlusion device of claim 1, wherein the structural component (102) comprises a coil.

3. The vascular occlusion device of claim 1, wherein the structural component (102) comprises a stent graft.

4. The vascular occlusion device of claim 1, wherein the sintered end (114, 116) extends an entirety of a distance between two opposing portions of an outer surface of the ePTFE membrane (104).

5. The vascular occlusion device of claim 1, further comprising one or more anchors (118) extending radially outward from the membrane (104).

6. The vascular occlusion device of claim 5, wherein the one or more anchors (118) comprises a portion of the structural component (102) extending through the membrane (104).

## Patentansprüche

1. Vaskuläre Okklusionsvorrichtung (100), umfassend:
eine strukturelle Komponente (102); und
eine Membran (104) aus expandiertem Polytetrafluorethylen (ePTFE), die die strukturelle Komponente (102) umgibt, wobei die ePTFE-Membran Folgendes umfasst:
eine im Wesentlichen zylindrisch geformte Röhre, die eine Wanddicke aufweist; und
ein gesintertes Ende (114, 116), das geschmolzenes und wieder verfestigtes ePTFE umfasst, wobei ein Abstand zwischen dem gesinterten Ende und einem Ende der strukturellen Komponente (102) vorhanden ist.

2. Vaskuläre Okklusionsvorrichtung nach Anspruch 1, wobei die strukturelle Komponente (102) eine Spule umfasst.

3. Vaskuläre Okklusionsvorrichtung nach Anspruch 1, wobei die strukturelle Komponente (102) einen Stentgraft umfasst.

4. Vaskuläre Okklusionsvorrichtung nach Anspruch 1, wobei sich das gesinterte Ende (114, 116) über einen gesamten Abstand zwischen zwei gegenüberliegenden Abschnitten einer Außenoberfläche der ePTFE-Membran (104) erstreckt.

5. Vaskuläre Okklusionsvorrichtung nach Anspruch 1, weiter umfassend einen oder mehrere Anker (118), die sich von der Membran (104) radial nach außen erstrecken.

6. Vaskuläre Okklusionsvorrichtung nach Anspruch 5, wobei der eine oder die mehreren Anker (118) einen Abschnitt der strukturellen Komponente (102) umfassen, der sich durch die Membran (104) erstreckt.

## Revendications

1. Dispositif d'occlusion vasculaire (100) comprenant :
un composant structural (102) ; et
une membrane en polytétrafluoroéthylène expansé (ePTFE) (104) entourant le composant structural (102), dans laquelle la membrane en ePTFE comprend :
un tube de forme sensiblement cylindrique ayant une épaisseur de paroi ; et
une extrémité frittée (114, 116) comprenant de l'ePTFE fondu et resolidifié, une distance étant présente entre l'extrémité frittée et une extrémité du composant structural (102).

2. Dispositif d'occlusion vasculaire selon la revendication 1, dans lequel le composant structural (102) comprend une spire.

3. Dispositif d'occlusion vasculaire selon la revendication 1, dans lequel le composant structurel (102) comprend un stent couvert.

4. Dispositif d'occlusion vasculaire selon la revendication 1, dans lequel l'extrémité frittée (114, 116) s'étend sur l'intégralité d'une distance entre deux portions opposées d'une surface externe de la membrane en ePTFE (104).

5. Dispositif d'occlusion vasculaire selon la revendication 1, comprenant en outre un ou plusieurs ancrages (118) s'étendant radialement vers l'extérieur à partir de la membrane (104).

6. Dispositif d'occlusion vasculaire selon la revendication 5, dans lequel le ou les ancrages (118) comprennent une portion du composant structural (102) s'étendant à travers la membrane (104).
